(19) **Europäisches Patentamt / European Patent Office / Office européen des brevets**

(11) **EP 1 788 052 B1**

(12) **EUROPEAN PATENT SPECIFICATION**

(45) Date of publication and mention
of the grant of the patent:
**16.11.2016 Bulletin 2016/46**

(51) Int Cl.:
***C09J 7/00*** *(2006.01)*

(21) Application number: **06019982.5**

(22) Date of filing: **25.09.2006**

(54) **Acrylic pressure sensitive adhesives**

Druckempfindliche Klebstoffe auf Acrylbasis

Adhésifs sensibles à la pression à base d'acryle

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **29.09.2005 US 238277**

(43) Date of publication of application:
**23.05.2007 Bulletin 2007/21**

(73) Proprietor: **Henkel AG & Co. KGaA
40589 Düsseldorf (DE)**

(72) Inventors:
• **Foreman, Paul B.
Somerville
New Jersey 08876 (US)**
• **Luciano, Allison
Lebanon
New Jersey 08833 (US)**

(56) References cited:
**WO-A-97/03144        WO-A2-02/062881
DE-B- 1 009 339       DE-B- 1 303 378
JP-A- 2001 049 217    US-A- 6 126 865**

**Description**

**FIELD OF THE INVENTION**

The invention relates to a solution of an acrylic pressure sensitive adhesive in a solvent having specifically defined solubility parameters.

**BACKGROUND OF THE INVENTION**

Solution acrylic pressure sensitive adhesives (PSAs) are conventionally used in the manufacture of pressure sensitive adhesive tapes, the adhesive tape comprising a backing and a PSA composition. <New page 1 a; A> One field where PSA compositions find wide spread use is the medical segment, e.g., various tapes, plasters, bandages and drug delivery devices. Transdermal drug delivery patches for example can be manufactured by preparing a coating formulation by mixing a solution of the adhesive in a solvent with the drug and any excipients to form a homogeneous solution or suspension; applying the formulation to a substrate (a backing or a release liner) using well known knife, roll or extrusion die coating methods; drying the coated substrate to remove the solvent; and laminating the exposed surface to a release liner or backing.

Acrylic pressure sensitive adhesives i.a. find widespread use as adhesives in electrical applications. For example, US 6,126,865 describes acrylic pressure sensitive adhesives based on acrylic acid esters of a monohydric alcohol having a homopolymer Tg of less than 0°C and a non-polar ethylenically unsaturated monomer having a homopolymer Tg of greater than 15°C and a solubility parameter of not greater than 10.5. The adhesives in addition comprise electrically conductive agents such as metal articles to provide them with electrical conductivity. The compositions are described as useful as vibration dampers in automobile applications. WO 97/03144 describes essentially the same adhesives for the application as vibration dampers.

An example of the use of acrylic polymers for use in medical applications in the prior art is WO 02/062881 A2 which describes a foam composition including a vehicle and surface modified nanoparticles disposed in the vehicle wherein the nanoparticles have a particle diameter of less than 100 nanometers. The vehicle may be constituted i.a. of acrylate pressure sensitive adhesives and is foamed once the nanoparticles have been dispersed therein. The foam is described as suitable for the manufacture of foamed personal care products such as bandages and wound dressings.

Organic solvents such as ethyl acetate are conventionally used to prepare solution pressures sensitive adhesive compositions. When used in the manufacture of transdermal patches, the active ingredients, e.g., drug, is dissolved in the solution. In order to achieve constant flux from a transdermal patch it is often necessary to load the adhesive matrix with a concentration of drug above its solubility limit, thereby maintaining a constant concentration gradient across the stratum corneum. When the drug is completely soluble in the adhesive solution it is likely to remain dissolved in the dried matrix as a supersaturated solution of drug in polymer. While this has sometimes been done by design in order to create a much larger concentration gradient, the flux decreases with time since there is no replacement reservoir of drug. Attempts to minimize the effects of depletion by having a large amount of drug, compared with the required dose, dissolved in a thick adhesive matrix result in wasteful, low utilization of the drug.

A major problem with supersaturation is that thermodynamics favors crystallization but the kinetics of this process is slow and unpredictable since nucleation is required. Thus patch developers may try to keep the drug dispersed during the formulating and drying steps. However, many drugs are too soluble in the adhesive's solvent system to permit easy dispersion.

There exists a need in the art for adhesive solutions based on acrylic polymers that overcome the above-described limitations. The currently invention addresses such need.

**SUMMARY OF THE INVENTION**

The invention provides an acrylic PSA in an organic solution in which the active ingredient (e.g., drugs) are dispersed rather than fully dissolved. When the active remains at least partially dispersed during the drying process, supersaturation is avoided.

One embodiment of this invention is directed to solution acrylic PSAs comprising a solvent or mixture of solvents which may include amounts of an aromatic hydrocarbon of not more than about 10 wt.-% on total solvents. The solvent or solvent mixture has a solubility parameter less than 17.8 but not less than 12, or, greater than or equal to 21.1 but not greater than 40 MPa$^{1/2}$. Preferably, the solubility parameter will be between about 14 and about 17.7 MPa$^{1/2}$ or between about 23 and about 30 MPa$^{1/2}$.

Another object of the invention is directed to solution acrylic PSAs comprising a solvent or mixture of solvents having a solubility parameter less than 17.1 but not less than 12, or, greater than about 21.1 but not greater than 40 MPa$^{1/2}$. Preferably, the solubility parameter will be between about 14 and 17 MPa$^{1/2}$ or between about 23 and 30 MPa$^{1/2}$.

Yet another embodiment of the invention provides articles manufactured using solution acrylic PSAs comprising a solvent or mixture of solvents having a solubility parameter as described above and having an active dispersed therein. The adhesive is well suited for the manufacture of articles such as tapes, plasters, bandages and transdermal drug delivery systems to be adhesively adhered to the skin. Acrylic polymers in high solubility parameter solvents are particularly well suited for delivering highly lipophilic drugs. Conversely, acrylic polymers in low solubility parameter solvents are particularly well suited for delivering more hydrophilic drugs.

A further embodiment is directed to a method of manufacturing articles comprising coating a backing substrate with a solution acrylic PSAs comprising a solvent or mixture of solvents having a solubility parameter as described above and having an active at least partially dispersed therein.

## BRIEF DESCRIPTION OF THE DRAWING FIGURE

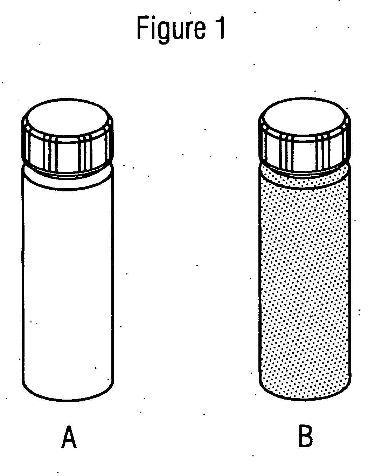

Figure 1 is photograph comparing the addition of ketoprofen at 15 wt. % on a solids basis to the polymer solutions of Comparative Example 2 (A) and Example 1 (B).

## DETAILED DESCRIPTION OF THE INVENTION

All references cited herein are incorporated in their entireties by reference.

The invention provides solution acrylic pressure sensitive adhesives that are suitable for dispersing actives which are soluble in conventional adhesive solutions. It has been discovered that solution acrylic PSAs prepared in solvents having a solubility parameter outside the conventional range permit the dispersion of a wider range of drugs than was heretofore possible and that these drugs remain dispersed during the drying process, thus avoiding supersaturation. In one embodiment the solution PSAs of the invention will comprise solvents or blends of solvents, which may include amounts of an aromatic hydrocarbon of not more than about 10 wt.-% on total solvents, having a solubility parameter less than 17.8 but not less than 12, or, greater than or equal to 21.1 but not greater than 40 MPa$^{\frac{1}{2}}$. In another embodiment of the invention, the solution acrylic PSAs of the invention will comprise a solvent or blend of solvents having a solubility parameter less than 17.1 but not less than 12, or, greater than about 21.1 but not greater than 40 MPa$^{\frac{1}{2}}$.

Table 1 lists the solubility parameter ($\delta$) of various solvents. The solvent blend solubility parameters of solvents were computed from published data on enthalpy of vaporization of the component solvents. Values for petroleum distillates such as commercial hexane or heptane were similarly computed from published data on their component isomers as determined by GC analysis.

The solubility parameter, $\delta$, of a component solvent is herein defined by the equation:

$$\delta = (E_{coh}/V_m)^{\frac{1}{2}} \qquad (1)$$

where $V_m$ is the molar volume and $E_{coh}$ is the cohesive energy density which is computed from the equation:

$$E_{coh} = \Delta H_{vap} - p\Delta V \approx \Delta H_{vap} - RT \qquad (2)$$

where $\Delta H_{vap}$ is the enthalpy of vaporization, R is the Universal Gas Constant and T is the absolute temperature. Values are calculated at T = 298K (25°C). Computation of solubility parameters is discussed in, for example, CRC Handbook of Solubility Parameters and Other Cohesion Parameters, A.F.M. Barton, CRC Press, New York (1983), the disclosure of which is incorporated herein in its entirety. It is noted that a number of semi-empirical, group contribution methods have been proposed for the calculation of solubility parameters. For the purposes of this invention, the values are computed from experimentally determined enthalpy data at 25°C as defined in equations (1) and (2). It can be shown (see Barton *ibid*) that the solubility parameter of a mixture of n solvents is given by the sum of $\delta_i$ for each of the individual component solvents weighted by their volume fraction, $v_i$:

$$\delta = \sum_{i=1}^{n} \delta_i v_i \qquad (3)$$

Conventional solution acrylic pressure sensitive adhesives for use in transdermal drug delivery are polymerized

in ethyl acetate ($\delta$ = 18.4 MPa$^{1/2}$) which has the advantage of being a pure solvent of low toxicity and sufficiently low boiling point to be readily removed in a drying oven. By design (to control molecular weight, for example), or because of a historical prior use in non-drug delivery applications, more complex solvent mixtures are commonly employed. Thus a typical conventional acrylic solution PSA may also contain hydrocarbon solvents and, if crosslinked with a metal alkoxide or chelate, stabilizing alcohols and ketones. Commercially available acrylic solution pressure sensitive adhesives for transdermal drug delivery applications employ solvent mixtures with solubility parameter falling within the range 17.1 $\leq \delta \leq$ 21.1 MPa$^{1/2}$. Due to increasing concern about the toxicity of aromatic hydrocarbon solvents such as toluene, solvent mixtures that are essentially free of aromatic hydrocarbons are preferred. Commercially available acrylic solution pressure sensitive adhesives that are free of aromatic hydrocarbons employ solvent mixtures with solubility parameter falling within the range 17.8 $\leq \delta <$ 21.1 MPa$^{1/2}$.

Solution pressure sensitive adhesives that employ a solvent or mixture of solvents having a solubility parameter less than 17.1 but not less than 12, or, greater than or equal to 21.1 but not greater than 40 MPa$^{1/2}$ are encompassed by the invention. In one embodiment, the solubility parameter will be between about 14 and about 17 MPa$^{1/2}$ or between about 23 and about 30 MPa$^{1/2}$.

Solution pressure sensitive adhesives that employ a solvent, or mixture of solvents having a solubility parameter less than 17.8 but not less than 12, or, greater than or equal to 21.1 but not greater than 40 MPa$^{1/2}$ and optionally comprising not more than about 10 wt.% on total solvent of aromatic hydrocarbons are also encompassed by the invention. In one embodiment, the solubility parameter will be between about 14 and about 17.7 MPa$^{1/2}$ or between about 23 and about 30 MPa$^{1/2}$.

Table 1

| Solvent | $\Delta$H (25°C) kJ/mol | B.Pt. °C | M. Wt. g/mol | $\rho$ g/cm$^3$ | $\delta$ MPa$^{1/2}$ |
|---|---|---|---|---|---|
| **C5 Aliphatic hydrocarbons** | | | | | |
| Cyclopentane | 28.52 | 49.3 | 70.13 | 0.7457 | 16.64 |
| n-Pentane | 26.43 | 36.06 | 72.15 | 0.6262 | 14.42 |
| 2-Methylbutane | 24.85 | 27.8 | 72.15 | 0.6201 | 13.87 |
| **C6 Aliphatic hydrocarbons** | | | | | |
| Cyclohexane | 33.01 | 80.73 | 84.16 | 0.7785 | 16.81 |
| Methylcyclopentane | 31.64 | 71.8 | 84.16 | 0.7486 | 16.11 |
| n-Hexane | 31.56 | 68.73 | 86.18 | 0.6548 | 14.86 |
| 2-Methylpentane | 29.89 | 60.26 | 86.18 | 0.6500 | 14.38 |
| 3-Methylpentane | 30.28 | 63.27 | 86.18 | 0.6598 | 14.59 |
| 2,2-Dimethylbutane | 27.68 | 43.73 | 86.18 | 0.6444 | 13.73 |
| 2,3-Dimethylbutane | 29.12 | 57.98 | 86.18 | 0.6616 | 14.30 |
| Hexane, commercial | | 66-69 | | 0.6680 | 14.96 |
| **C7 Aliphatic hydrocarbons** | | | | | |
| Methylcyclohexane | 35.36 | 100.93 | 98.19 | 0.7694 | 16.05 |
| Ethylcyclopentane | 36.40 | 103.5 | 98.19 | 0.7665 | 16.27 |
| cis-1,3-Dimethylcyclopentane | 34.20 | 90.8 | 98.19 | 0.7402 | 15.46 |
| n-Heptane | 36.57 | 98.5 | 100.20 | 0.6837 | 15.25 |
| 2-Methylhexane | 34.87 | 90.04 | 100.20 | 0.6787 | 14.81 |
| 3-Methylhexane | 35.06 | 91.84 | 100.20 | 0.6860 | 14.94 |
| 2,2-Dimethylpentane | 32.42 | 79.2 | 100.20 | 0.6739 | 14.19 |
| 2,3-Dimethylpentane | 34.26 | 89.78 | 100.20 | 0.6951 | 14.85 |
| 2,4-Dimethylpentane | 32.88 | 80.49 | 100.20 | 0.6727 | 14.29 |
| 3,3-Dimethylpentane | 33.03 | 86.06 | 100.20 | 0.6936 | 14.54 |

(continued)

| | | | | | |
|---|---|---|---|---|---|
| **C7 Aliphatic hydrocarbons** | | | | | |
| 3-Ethylpentane | 35.22 | 93.5 | 100.20 | 0.6982 | 15.10 |
| 2,2,3-Trimethylbutane | 32.05 | 80.86 | 100.20 | 0.6901 | 14.27 |
| Heptane, commercial | | 89-92 | | 0.6856 | 14.88 |
| **Aromatic Hydrocarbons** | | | | | |
| Toluene | 38.01 | 110.63 | 92.14 | 0.8669 | 18.28 |
| o-Xylene | 43.43 | 144.5 | 106.17 | 0.8802 | 18.43 |
| m-Xylene | 42.65 | 139.1 | 106.17 | 0.8642 | 18.08 |
| p-Xylene | 42.40 | 138.4 | 106.17 | 0.8611 | 17.99 |
| Ethylbenzene | 42.24 | 136.19 | 106.17 | 0.8670 | 18.02 |
| Xylene, commercial | | | | 0.8677 | 18.14 |
| Esters | | | | | |
| Ethyl acetate | 35.60 | 77.11 | 88.11 | 0.9003 | 18.40 |
| **Ketones** | | | | | |
| Acetone | 30.99 | 56.05 | 58.08 | 0.7899 | 19.69 |
| Methylethylketone | 34.79 | 79.59 | 72.11 | 0.8054 | 19.00 |
| **Alcohols** | | | | | |
| Methanol | 37.43 | 64.6 | 32.04 | 0.7914 | 29.38 |
| Ethanol | 42.32 | 78.4 | 46.07 | 0.7893 | 26.13 |
| i-Propanol | 45.39 | 82.3 | 60.10 | 0.7855 | 23.68 |
| Data source: Handbook of Chemistry and Physics, 78th ed., David R. Lide ed., CRC Press, New York (1997-8) | | | | | |

The acrylic polymer used to prepare the pressure sensitive adhesive is not particularly limiting as long as the acrylic polymer is pressure sensitive. As used herein, the term "pressure-sensitive adhesive" refers to a viscoelastic material which adheres instantaneously to most substrates with the application of slight pressure and remains permanently tacky. A polymer is a pressure-sensitive adhesive within the meaning of the term as used herein if it has the properties of a pressure-sensitive adhesive per se or functions as a pressure-sensitive adhesive by admixture with tackifiers, plasticizers or other additives.

The acrylic polymer comprises at least one low glass transition temperature (Tg) alkyl acrylate monomer. Low Tg monomers are those having a homopolymer Tg of less than about 0°C. Preferred alkyl acrylates which may be used to practice the invention have up to about 18 carbon atoms in the alkyl group, preferably from about 4 to about 12 carbon atoms in the alkyl group. Alkyl acrylates for use in the invention include methyl acrylate, butyl acrylate, amyl acrylate, hexyl acrylate, 2-ethylhexyl acrylate, octyl acrylate, isooctyl acrylate, decyl acrylate, dodecyl acrylates, isomers thereof, and combinations thereof. Particularly preferred are butyl acrylate, 2-ethylhexyl acrylate and/or isooctyl acrylate, most preferably 2-ethylhexyl acrylate.

The acrylic polymer may also comprise one or more vinyl ester monomers, particularly preferred is vinyl acetate, and/or may comprise one or more functional monomers. Preferred are carboxy and/or hydroxy functional monomers. Useful carboxylic acids preferably contain from about 3 to about 6 carbon atoms and include, among others, acrylic acid, methacrylic acid, itaconic acid, $\beta$-carboxyethyl acrylate and the like. Acrylic acid, methacrylic acid and mixtures thereof are particularly preferred. Examples of hydroxy functional monomers include hydroxyethyl acrylate, hydroxypropyl acrylate, hydroxyethyl methacrylate and hydroxypropyl methacrylate. Preferred for use is hydroxyethyl acrylate.

The adhesives may also contain a nitrogen containing compound, in particularly N-substituted acrylamides or methacrylamides. Examples include N-vinyl pyrrolidone, N-vinyl caprolactam, N-tertiary octyl acrylamide (t-octyl acrylamide), dimethyl acrylamide, diacetone acrylamide, N-tertiary butyl acrylamide, N-isopropyl acrylamide, N-vinyl acetamide and/or N-vinyl formamide.

The acrylic polymer may optionally further comprise other well known comonomers including monomers having a high glass transition temperature (i.e., a Tg greater than about 0°C). Non-limiting examples include methyl acrylate, methyl methacrylate, ethyl acrylate and/or isobutyl methacrylate. Other comonomers can be used to modify the Tg of the acrylic polymer. Such comonomers include styrene, amides such as acrylamide or methacrylamide, and/or nitriles such as acrylonitrile or cyanoethylacrylate.

Minor amounts, sufficient to increase cohesion without gelling the solution, of crosslinkable multifunctional monomers may be used including, for example, glycidyl methacrylate, allyl glycidyl ether, hexanedioldi(meth)acrylate and the like.

The adhesive compositions of the present invention may also optionally include other monomers such as amines, for example 2-(diethylamino)ethyl methacrylate, to impart functionality to the adhesive or for the purposes of compatibility with a particular drug or excipient.

Adhesives of the invention may also comprise blended polymers wherein the acrylic polymer is blended with and further comprises other types of polymers, including silicone polymers such as polydimethylsiloxane and polymethylphenylsiloxane and rubber polymers such as polyiso-butylene and styrene-isoprene-styrene block copolymer.

In addition to the acrylic polymers, or blends thereof, the adhesive compositions of the invention may optionally comprise a compatible tackifier and/or plasticizer and/or skin permeation enhancers. The acrylic polymer and any tackifier, plasticizer and/or permeation enhancer will be selected and used in amounts effective to produce the desired properties required for the intended end use.

The adhesive compositions may if desired be formulated with a crosslinking agent. Use of a crosslinker adds to the cohesive strength. Preferred are chemical crosslinking agents. In particular, crosslinking agents containing aluminum or titanium may be used to practice the invention. Non-limiting examples include aluminum tris(acetylacetonate) and bis (2,4-pentanedionate-O,O') bis (2-propanolato) titanium. The crosslinker is typically added in an amount of from about 0.3% to about 2% by weight of the acrylic copolymer.

The compositions of the invention may include other additives known to those skilled in the art to satisfy different properties and meet specific application requirements. Such additives include, for example, antioxidants, fillers, pigments, rheology modifiers, which may be incorporated in minor or larger amounts into the adhesive formulation, depending on the purpose.

The adhesives are useful for delivering drugs through the skin (transdermal) or delivering actives to the skin (dermal). The delivery process may be aided by including a permeation enhancer in the adhesive composition. The level of enhancer in the final solid adhesive is determined by the desired flux of active and may be limited by the requirements for resistance to cold flow. Enhancer loadings up to about 30% on adhesive polymer solids, more typically from about 5 to about 15% may be employed.

It will be obvious to one skilled in the art that the acrylic pressure sensitive adhesive polymer composition needs to be designed such that good pressure sensitive adhesive properties are achieved in the final formulation taking into account all added components including the active therapeutic agent, and enhancers, plasticizers, crosslinkers, tackifiers and/or other excipients, if present. It will be further obvious that such excipients may change the solubility parameter of the liquid vehicle for the polymer and that this will need to be taken into account when considering the ability to at least partially disperse a drug in the solution. Partial dispersion, as used herein, means that, upon mixing with the adhesive solution, only a portion of the active ingredient goes into solution, the remainder being suspended as solid particles within the solution. Similarly, after drying the term partial dispersion is intended to mean that a portion of the active is dissolved in the adhesive matrix and the remainder is distributed within the adhesive matrix as solid particles.

The adhesive may be advantageously formulated for use in, for example, medical applications such as wound care, transdermal or dermal drug or cosmeceutical delivery applications. The term transdermal refers to the use of the skin as a portal for the administration of drugs by topical application. The topically applied drug passes into and/or through the skin. Thus "transdermal" is used broadly to refer to the topical administration of a drug which acts locally, i.e., at the surface or within the skin, such as, for example, a blemish patch used to treat acne, and to the topical application of a drug which acts systemically by diffusing through the skin and entering the blood stream.

The pressure sensitive adhesives of the invention having an active dispersed therein may advantageously be used in the manufacture of adhesive articles including, but not limited to, medical tapes and transdermal drug delivery systems including transdermal drug-delivery patches, diagnostic patches, dermal patches for delivery of skin actives and patches for providing a skin care function.

In one embodiment, the adhesive article comprises an adhesive coated on at least one major surface of a backing having a first and second major surface. The adhesive may be present on one or both surfaces of the backing. When the adhesive is coated on both surfaces of the backing, the adhesive on each surface can be the same or different. One embodiment is directed to a transdermal drug delivery system comprising an adhesive layer containing an at least partially dispersed therapeutic agent and a backing layer. In another embodiment, the drug delivery system also comprises a release layer. When the patient peels the release liner from the adhesive and applies the patch, the drug partitions into the stratum corneum (outer skin layer) and permeates through the epidermis and dermis.

Backings which can be used in the practice of this invention include, with or without modification, metal foils, metalized polyfoils, composite foils or films containing poytetrafluoroethylene (TEFLON®)-type materials or equivalents thereof, polyether block amide copolymers, polyurethanes, polyvinylidene chloride, nylon, silicone elastomers, rubber-based poylisobutylene styrene, styrenebutadiene and styrene-isoprene copolymers, polyethylene, polyester, and other such materials used in the art of transdermal drug delivery. Particularly preferred are thermoplastic polymers such as polyolefins, for example polyethylene and polypropylene, and polyesters such as polyethyleneterephthalate.

The term "drug" is to be construed herein in its broadest sense to mean any agent which is intended to produce some therapeutic benefit. The agent may or may not be pharmaceutically active, but will be "bioactive" in the sense that it has an effect on the human body. The agent may be used to treat or alter a condition, which may or may not be a pathological, i.e., a disease state. "Drug", "bioactive agent," "preparation," "medicament," "therapeutic agent," "physiological agent" and "pharmaceutical agent" are used interchangeably herein and include substances for use in the diagnosis, cure, mitigation, arrest, treatment or prevention of a condition or disease state or to affect the structure or function of the body. Skin-wellness agents that function to e.g., soften and moisturize are included in this term. The term "treatment" is used broadly to encompass prevention, alteration, cure and control of the condition.

The drug is present in a drug delivery device of the invention in a therapeutically effective amount, i.e., an amount effective to bring about a desired therapeutic result in the treatment of a condition to which the preparation of this invention is to be applied. Effective amount of a drug means a nontoxic but sufficient amount of a drug to provide the selected effect over a specific period of time. The amount that constitutes a therapeutically effective amount varies according to the particular drug incorporated in the device, the condition being treated, any drugs being co-administered with the selected drug, desired duration of treatment, the surface area of the skin over which the device is to be placed, and other components of the drug delivery device. Such an amount is readily determinable by the skilled practitioner.

The drug delivery system of the invention, in addition to the drug, may advantageously also contain an effective amount of a penetration enhancer. An effective amount of a penetration enhancer means an amount that provides a selected increase in membrane permeability, rate of administration and amount of drug.

The device of the invention is placed on the skin and allowed to remain for a time sufficient to achieve or maintain the intended therapeutic effect. The time that constitutes a sufficient time can be selected by those skilled in the art with consideration of the flux rate of the device of the invention and of the condition being treated.

Treatment areas where the delivery device of the invention finds use, and pharmaceutical products which are incorporated into the devices of the invention, are selected form treatment for incontinence (oxybutinin), central nervous system conditions (methylphenidate), hormone therapy and birth control (estradiol, testosterone, progestin, progesterone, levonorgestrel) cardiovascular (nitroglycerin, clonidine) and cardiotonics (e.g., digitalis, digoxin), pain management or anti-inflammatory (fentanyl, lidocaine, diclofenac, flurbiprofen), cosmetic (benzoyl peroxide, salicylic acid, vitamin C, vitamin E, aromatic oils), antinauseants (scopalamine), smoking cessation (nicotine), antiinflammatory conditions, both steroidal (e.g., hydrocortisone, prednisolone, triamcinolone) and nonsteroidal (e.g., naproxen, piroxicam) treatments, antibacterials (e.g., penicillins such as penicillin V, cephalosporins such as cephalexin, erythromycin, tetracycline, gentamycin, sulfathiazole, nitrofurantoin, and quinolones such as norfloxacin, flumequine, and ibafloxacin), antiprotazoals (e.g., metronidazole), antifungals (e.g. nystatin), calcium channel blockers (e.g. nifedipine, diltiazem), bronchodilators (e.g., theophylline, pirbuterol, salmeterol, isoproterenol), enzyme inhibitors such as collagenase inhibitors, protease inhibitors, elastase inhibitors, lipoxygenase inhibitors, and angiotensin converting enzyme inhibitors (e.g., captopril, lisinopril), other antihypertensives (e.g., propranolol), leukotriene antagonists, anti-ulceratives such as H2 antagonists, antivirals and/or immunomodulators (e.g., 1-isobutyl-1H-imidazo[4,5-c]quinolin-4-amine, 1-(2-hydroxy-2-methylpropyl)-1H-imidazo[4,5-c]quinoline-4-amine, and acyclovir), local anesthetics (e.g., benzocaine, propofol), antitussives (e.g., codeine, dextromethorphan), antihistamines (e.g., diphenhydramine, chlorpheniramine, terfenadine), narcotic analgesics (e.g., morphine, fentanyl), cardioactive products such as atriopeptides, anticonvulsants (e.g., carbamazine), immunosuppressives (e.g., cyclosporine), psychotherapeutics (e.g., diazepam), sedatives (e.g., phenobarbital), anticoagulants (e.g., heparin), analgesics (e.g., acetaminophen), antimigrane agents (e.g., ergotamine, melatonin, sumatriptan), antiarrhythmic agents (e.g., flecainide), antiemetics (e.g., metaclopromide, ondansetron), anticancer agents (e.g., methotrexate), neurologic agents such as anxiolytic drugs, hemostatics, anti-obesity agents, as well as pharmaceutically acceptable salts, esters, solvates and clathrates thereof.

Disclosed is further that veterinary drugs may also be conveniently applied using the transdermal drug delivery device as described above, as well as agricultural and horticultural agents. It will be appreciated that transdermal drug delivery in veterinary and horticultural applications enables more exact dosing, and less waste than administration in the food/irrigation water.

The transdermal delivery devices of the invention can be made in the form of an article such as a tape, a patch, a sheet, a dressing or any other form known to those skilled in the art. The dosage system may be produced in any desirable unit form. A circular form is convenient as it contains no corners which might be easily detached from the skin. In addition to having various shapes, the dosage units produced may come in various sizes.

Depending on the design of the patch and the condition to be treated, the patch will remain on the skin for up to an hour or more, up to about one week. In a preferred embodiment, the patch is designed to remain on the skin at the application site for about 24 hours, and to be changed daily. In another preferred embodiment, the patch is to remain on the skin and be changed from once to twice a week. Preferably, the patch will be placed on the skin at a site different from the location of the previously used patches.

The invention will be described further in the following examples, which are included for purposes of illustration.

## EXAMPLES

### Example 1

An initial charge containing 32.1 g 2-ethylhexylacrylate, 13.7 g methylacrylate, 3.6 g acrylic acid, 82.5 g cyclohexane, and 0.17 g 2,2'-azobisisobutyronitrile (AIBN) polymerization initiator was prepared and charged to a two liter 4-neck round bottomed flask equipped with stainless steel stirrer, thermometer, condenser, water bath, and slow addition funnels. The initial charge was heated to reflux temperature while stirring. At 10 minutes following the beginning of reflux, a monomer mixture containing 182.5 g 2-ethylhexylacrylate, 77.1 g methylacrylate and 21.0 g acrylic acid and 181.50 g cyclohexane was added uniformly over a period of 2 hours. Also beginning at 10 minutes from the start of reflux, a mixture of 168.3 g cyclohexane and 0.79 g AIBN predissolved in 6.6 g ethyl acetate was added uniformly over a period of 3 hours. Reflux was maintained for a further 2 hours. A solution of 2.5 g of a short half-life polymerization initiator in 72.6 g cyclohexane was then added uniformly over a period of one hour and the flask contents maintained at reflux temperature for a further 2 hours in order to scavenge residual monomers. At the end of the hold period, the flask contents were diluted with 231.1 g cyclohexane, cooled to room temperature and the pressure sensitive adhesive solution discharged. The adhesive solution had a solids content of 37.2 %, Brookfield viscosity 26,000 mPa.s and exhibited a slightly hazy appearance. The solubility parameter of the solvent system was calculated from the data in Table 1 to be 16.8 MPa$^{1/2}$.

### Example 2

### (comparative example)

The adhesive of Example 1 was polymerized in a mixture of ethyl acetate and commercial grade hexane in a 78:22 % weight ratio and diluted with a mixture of ethyl acetate, isopropanol and toluene to give an overall solvent blend consisting of 65 % ethyl acetate, 12 % hexane, 19% isopropanol and 2 % toluene by weight. The adhesive solution had a solids content of 34.0 %, Brookfield viscosity 2,150 mPa.s and was clear and colorless. The solubility parameter of the solvent system was calculated from the data in Table 1 to be 19.0 MPa$^{1/2}$.

### Example 3

Ketoprofen was added to the adhesive solution of Examples 1 and 2 at 15% on a solids basis. The samples were mixed overnight. As can be seen in Figure 1, the ketoprofen completely dissolved in the adhesive solution of Example 2 (tube A) whereas in the Example 1 solution (tube B) most of the ketoprofen remained as a dispersed solid.

Adhesive films were prepared by drying for 30 minutes at 60°C the solutions containing the drug. The film prepared from the cyclohexane adhesive solution (Example 1) contained undissolved crystals of ketoprofen even after drying whereas the film prepared from the conventional adhesive solution (Comparative Example 2) showed no visible crystals indicating that the dissolved drug remains in supersaturated state after drying. This was confirmed by Differential Scanning Calorimetry (DSC) showing zero enthalpy of fusion. In subsequent experiments dried films from Example 1 polymer solution were prepared in which the concentration of ketoprofen was reduced until the enthalpy of fusion of ketoprofen crystals, determined by DSC, approached zero. These measurements placed an upper limit of 2.5 wt.% on the solubility of ketoprofen in the dried adhesive film.

### Example 4

The adhesive of Example 1 was polymerized in commercial grade heptane ($\delta$=14.9 MPa$^{1/2}$) substituting for cyclohexane and yielded a very hazy solution.

### Example 5

An initial charge containing 69.0 g 2-ethylhexylacrylate, 65.1 g n-butylacrylate, 60.0 g t-octylacrylamide 30.0 g methylmethacrylate, 187.4 g cyclohexane, and 0.15 g lauroyl peroxide polymerization initiator was prepared and charged to a two liter 4-neck round bottomed flask equipped with stainless steel stirrer, thermometer, condenser, water bath, and slow addition funnels. The initial charge was heated to reflux temperature while stirring. At 45 minutes following the beginning of reflux, a monomer mixture containing 28.5 g 2-ethylhexylacrylate, 32.4 g n-butylacrylate, 15.0 g methyl-methacrylate was added uniformly over a period of 1 hour. Also beginning at 45 minutes from the start of reflux, a mixture of 41.6 g cyclohexane and 1.08 g lauroyl peroxide was added uniformly over a period of 2 hours. Beginning 190 minutes after initial reflux 107.4 g cyclohexane was uniformly added over 3 hours and the solution was then held at reflux temperature for 6.5 hours. A solution of 1.6 g of a short half-life polymerization initiator in 30.0 g cyclohexane was then added uniformly over a period of one hour and the flask contents maintained at reflux temperature for a further 1 hour in order to scavenge residual monomers. At the end of the hold period, the flask contents were diluted with 16.7 g cyclohexane, cooled to room temperature and the pressure sensitive adhesive solution discharged. The adhesive solution had a solids content of 44.0 %, Brookfield viscosity 8,300 mPa.s and exhibited a slightly hazy, colorless appearance. The solubility parameter of the purely aliphatic hydrocarbon solvent was 16.8 MPa$^{1/2}$.

### Example 6

The polymer of Example 5 was prepared using methylcyclopentane (($\delta$=16.1 MPa$^{1/2}$) in place of cyclohexane. A colorless adhesive solution, solids 44.3%, viscosity 890 mPa.s, was obtained having a slightly hazy appearance.

### Example 7

An initial charge containing 52.0 g n-butylacrylate, 24.8 g 2-hydroxyethylacrylate, 3.30 g 2-ethylhexylacrylate, 2.5 g methylmethacrylate, 138.6 g ethanol (solvent), and 0.17 g AIBN was prepared and charged to a 2-L 4-neck round bottom flask equipped with stainless steel stirrer, thermometer, condenser, water bath, and slow addition funnels. The initial charge was heated to reflux while stirring. At 15 minutes from the start of reflux, monomer mix containing 155.9 g n-butyl acrylate, 74.3 g 2-hydroxyethylacrylate, 9.9 g 2-ethylhexylacrylate, 7.4 g methylmethacrylate, and 181.5 g ethanol were simultaneously and uniformly added over a period of 2 hours. Also at 15 minutes from the start of reflux, 51.2 g ethanol and 1.98 g AIBN were simultaneously and uniformly added over a period of 4 hours. At the end of the addition, the flask contents were held at reflux for 1 hour. At 315 minutes from the start of reflux, a solution of 1.2 g of a short half-life initiator in 9.3 g ethanol was added over a period of 1 hour. At the end of the addition, the flask contents were held at reflux for 1 hour. At the end of the hold period, the contents were cooled to room temperature, diluted with 104.0 g ethanol and the solution of pressure sensitive adhesive discharged. A low viscosity, colorless, slightly hazy solution resulted, having a solids content of 40.0%.

### Example 8

### (comparative example)

The polymer of Example 8 was prepared substituting ethyl acetate for ethanol in all steps except the dilution which consisted of 47.3 g ethyl acetate, 37.8 g isopropanol and 18.9 g methanol. The pressure sensitive adhesive solution had solids content of 39.9%, viscosity of 1,200 mPa.s and had a clear, very slightly yellow appearance. The overall solvent mixture was calculated to have solubility parameter $\delta$ = 19.4 MPa$^{1/2}$.

### Example 9

Cholesteryl dodecanoate, a model for a highly lipophilic drug, was added at 1% on a solids basis to the solutions of Example 7 and 8. These were then mixed overnight at room temperature. The solution of Example 7 contained a large quantity of undissolved model active whereas in the solution of Example 8 the model active was completely soluble.

## Claims

1. A solution of a pressure sensitive adhesive comprising an acrylic pressure sensitive polymer, a solvent or mixture of solvents, said solvent or mixture of solvents having a solubility parameter of

   a) less than 17.1 but not less than 12, or, greater than or equal to 21.1 but not greater than 40 MPa$^{1/2}$, or
   b) a solubility parameter of less than 17.8, but not less than 12, or greater than or equal to 21.1 but not greater than 40 MPa$^{1/2}$ and optionally an aromatic hydrocarbon, said aromatic hydrocarbon being present at not more than about 10 wt.-% on total solvents,

   and an active ingredient, wherein the solubility parameter $\delta$ is calculated as $\delta = (E_{coh}/V_m)^{1/2}$ where $V_m$ is the molar

volume and $E_{coh}$ is the cohesive energy density which is computed from the equation $E_{coh} = \Delta H_{vap} - p\Delta V \approx \Delta H_{vap} - RT$ where $\Delta H_{vap}$ is the enthalpy of vaporization, R is the Universal Gas Constant and T is the absolute temperature, and wherein the solubility parameters are calculated from $\Delta H$ at 25 °C, the acrylic pressure sensitive adhesive polymer comprises an alkyl acrylate monomer having a homopolymer Tg of less than 0°C, which has up to 18 carbon atoms in the alkyl group and wherein the active ingredient is selected from oxybutinin, methylphenidate, estradiol, testosterone, progestin, progesterone, levonorgestrel, nitroglycerin, clonidine, pain management or anti-inflammatory agents, benzoyl peroxide, salicylic acid, vitamin C, vitamin E, aromatic oils, antinauseants, nicotine, antiinflammatory agents including hydrocortisone, prednisolone, triamcinolone, naproxen and piroxicam, antibacterials, antiprotazoals, antifungals, calcium channel blockers, bronchodilators, collagenase inhibitors, protease inhibitors, elastase inhibitors, lipoxygenase inhibitors, and angiotensin converting enzyme inhibitors, antihypertensives, leukotriene antagonists, anti-ulceratives, antivirals and/or immunomodulators, local anesthetics, antitussives, antihistamines, narcotic analgesics, atriopeptides, anticonvulsants, immunosuppressives, psychotherapeutics, sedatives, anticoagulants, analgesics, antimigrane agents, antiarrhythmic agents, antiemetics, anticancer agents, neurologic agents, hemostatics, anti-obesity agents, and pharmaceutically acceptable salts, esters, solvates and clathrates thereof.

2. The solution of embodiment a) of claim 1 wherein the solubility parameter is between 14 and about 17 MPa$^{½}$ or between about 23 and 30 MP$^{½}$.

3. The solution of embodiment b) of claim 1 wherein the solubility parameter is between about 14 and about 17.7 MPa$^{½}$ or between about 23 and 30 MPa$^{½}$.

4. The solution of claim 1 wherein the solvent or solvent mixture comprises cyclohexane.

5. The solution of claim 1 wherein the active ingredient is at least partially dispersed in said solution.

6. An article manufactured using the solution of claim 1.

7. The article of claim 6 which is a medical tape.

8. The article of claim 6 which is a drug delivery device.

9. The article of claim 8 wherein the drug is at least partially dispersed within the adhesive matrix.

10. A method of manufacturing an article to be adhesively adhered to the skin comprising coating a backing substrate with the solution acrylic pressure sensitive adhesive of claim 4 and drying the solution.

**Patentansprüche**

1. Lösung eines Haftklebstoffs, Folgendes umfassend: ein druckempfindliches Acrylpolymer, ein Lösungsmittel oder eine Mischung aus Lösungsmitteln, wobei das Lösungsmittel oder die Mischung aus Lösungsmitteln Folgendes aufweist: einen Löslichkeitsparameter, der

a) kleiner als 17,1, aber nicht kleiner als 12, oder größer als oder gleich 21,1, aber nicht größer als 40 MPa$^{½}$ ist, oder
b) einen Löslichkeitsparameter, der kleiner als 17,8, aber nicht kleiner als 12, oder größer als oder gleich 21,1, aber nicht größer als 40 MPa$^{½}$ ist, und optional einen aromatischen Kohlenwasserstoff, wobei der Kohlenwasserstoff mit nicht mehr als etwa 10 Gew.-% bezogen auf die gesamten Lösungsmittel vorhanden ist,

und einen Wirkstoff, wobei der Löslichkeitsparameter $\delta$ als $\delta = (E_{coh}/V_m)^{½}$ berechnet wird, wobei $V_m$ das Molvolumen und $E_{coh}$ die Kohäsionsenergiedichte ist, die aus der Gleichung $E_{coh} = \Delta H_{vap} - p\Delta V \approx \Delta H_{vap} - RT$ berechnet wird, wobei $\Delta H_{vap}$ die Verdampfungsenthalpie, R die universelle Gaskonstante und T die absolute Temperatur ist und wobei die Löslichkeitsparameter aus $\Delta H$ bei 25°C berechnet werden, wobei das Acrylhaftklebstoffpolymer ein Alkylacrylatmonomer mit einer Homopolymer-Tg von weniger als 0 °C, das bis zu 18 Kohlenstoffatome in der Alkylgruppe aufweist, umfasst und wobei der Wirkstoff ausgewählt ist aus Oxybutynin, Methylphenidat, Estradiol, Testosteron, Progestin, Progesteron, Levonorgestrel, Nitroglycerin, Clonidin, Schmerzbehandlungs- oder entzündungshemmenden Mitteln, Benzoylperoxid, Salicylsäure, Vitamin C, Vitamin E, aromatischen Ölen, Mitteln gegen Übelkeit,

**EP 1 788 052 B1**

Nikotin, entzündungshemmenden Mitteln einschließlich Hydrocortison, Prednisolon, Triamcinolon, Naproxen und Piroxicam, antibakteriellen Mitteln, Antiprotozoika, Antimyotika, Calciumkanalblockern, Bronchodilatatoren, Collagenasehemmern, Proteasehemmern, Elastasehemmern, Lipoxygenasehemmern und Angiotensin-Konversionsenzym-Hemmern, Antihypertensiva, Leukotrienantagonisten, Antiulcusmitteln, antiviralen Mitteln und/oder Immunmodulatoren, Lokalanästhetika, Antitussiva, Antihistaminika, Betäubungsmitteln, Atriopeptiden, Antikonvulsiva, Immunsuppressiva, Psychotherapeutika, Sedativa, Antikoagulantien, Analgetika, Migränemitteln, Antiarrhythmika, Antiemetika, Krebsmitteln, neurologischen Mitteln, Hämostatika, Adipositasmitteln sowie pharmazeutisch unbedenklichen Salzen, Estern, Solvaten und Clathraten davon.

2. Lösung nach Ausführungsform a) von Anspruch 1, wobei der Löslichkeitsparameter zwischen 14 und etwa 17 MPa$^{1/2}$ oder zwischen etwa 23 und 30 MP$^{1/2}$ liegt.

3. Lösung nach Ausführungsform b) von Anspruch 1, wobei der Löslichkeitsparameter zwischen etwa 14 und etwa 17,7 MPa$^{1/2}$ oder zwischen etwa 23 und 30 MPa$^{1/2}$ liegt.

4. Lösung nach Anspruch 1, wobei das Lösungsmittel oder das Lösungsmittelgemisch Cyclohexan umfasst.

5. Lösung nach Anspruch 1, wobei der Wirkstoff wenigstens teilweise in der Lösung dispergiert ist.

6. Erzeugnis, das unter Verwendung der Lösung nach Anspruch 1 hergestellt wurde.

7. Erzeugnis nach Anspruch 6, wobei es sich um ein medizinisches Klebeband handelt.

8. Erzeugnis nach Anspruch 6, wobei es sich um eine Arzneimittelabgabevorrichtung handelt.

9. Erzeugnis nach Anspruch 8, wobei das Arzneimittel wenigstens teilweise in der Klebstoffmatrix dispergiert ist.

10. Verfahren zum Herstellen eines haftend an die Haut zu klebenden Erzeugnisses, umfassend Beschichten eines Trägersubstrats mit der Lösung eines Acrylhaftklebstoffs nach Anspruch 4 und Trocknen der Lösung.

**Revendications**

1. Solution d'un adhésif sensible à la pression comprenant un polymère acrylique sensible à la pression, un solvant ou mélange de solvants, ledit solvant ou mélange de solvants ayant un paramètre de solubilité de

a. moins de 17,1 mais pas moins de 12, ou supérieur ou égal à 21,1 mais non supérieur à 40 Mpa$^{1/2}$, ou
b. un paramètre de solubilité de moins de 17,8, mais de pas moins de 12, ou supérieur ou égal à 21,1 mais non supérieur à 40 MPa$^{1/2}$ et éventuellement un hydrocarbure aromatique, l'hydrocarbure aromatique étant présent à pas plus de 10% en poids environ sur les solvants totaux,

et un ingrédient actif, le paramètre de solubilité $\delta$ étant calculé comme ceci $\delta = (E_{coh}/Vm)^{1/2}$ où $V_m$ est le volume molaire et $E_{coh}$ est la densité d'énergie de cohésion, qui est calculée à partir de l'équation $E_{coh} = \Delta H_{vap} - p\Delta V \approx \Delta H_{vap} - RT$ où $\Delta H_{vap}$ est l'enthalpie de vaporisation, R est la Constante Universelle des Gaz et T est la température absolue, et les paramètres de solubilité étant calculés à partir de $\Delta H$ à 25°C, le polymère acrylique adhésif sensible à la pression comprenant un monomère d'acrylate d'alkyle ayant un homopolymère Tg de moins de 0°C, qui a jusqu'à 18 atomes de carbone dans le groupe alkyle et l'ingrédient actif étant choisi parmi l'oxybutinine, le méthylphénidate, l'oestradiol, la testostérone, la progestine, la progestérone, le lévonorgestrel, la nitroglycérine, la clonidine, les agents de gestion de la douleur ou anti-inflammatoire, le peroxyde de benzoyle, l'acide salicylique, la vitamine C, la vitamine E, les huiles aromatiques, les antinauséeux, la nicotine, les agents anti-inflammatoires incluant l'hydrocortisone, la prednisolone, la triamcinolone, le naproxène et le piroxicam, les antibactériens, les anti-protozoaires, les antifongiques, les inhibiteurs calciques, les bronchodilatateurs, les inhibiteurs de collagénase, les inhibiteurs de protéase, les inhibiteurs d'élastase, les inhibiteurs de lipoxygénase et les inhibiteurs de l'enzyme de conversion de l'angiotensine, les antihypertenseurs, les antagonistes des leucotriènes, les antiulcéreux, les antiviraux et/ou les immunomodulateurs, les anesthésiques locaux, les antitussifs, les antihistaminiques, les analgésiques narcotiques, les atriopeptides, les anticonvulsivants, les immunosuppresseurs, les psychothérapeutiques, les sédatifs, les anticoagulants, les analgésiques, les agents antimigraineux les agents anti-arythmiques, les antiémétiques, les agents anticancéreux, les agents neurologiques, les hémostatiques, les agents anti-obésité et leurs sels, esters, solvates et clathrates pharmaceutiquement acceptables.

2. Solution du mode de réalisation a) selon la revendication 1, dans laquelle le paramètre de solubilité est compris entre 14 et environ 17 MPa$^{1/2}$ ou entre 23 et 30 MP$^{1/2}$ environ.

3. Solution du mode de réalisation b) selon la revendication 1, dans laquelle le paramètre de solubilité est compris entre environ 14 et environ 17,7 MPa$^{1/2}$ ou entre environ 23 et 30 MPa$^{1/2}$.

4. Solution selon la revendication 1, dans laquelle le solvant ou mélange de solvants comprend le cyclohexane.

5. Solution selon la revendication 1, dans laquelle l'ingrédient actif est au moins partiellement dispersé dans ladite solution.

6. Article fabriqué avec la solution selon la revendication 1.

7. Article selon la revendication 6, qui est un ruban médical.

8. Article selon la revendication 6, qui est un dispositif de délivrance de médicament.

9. Article selon la revendication 8, dans lequel le médicament est au moins partiellement dispersé à l'intérieur de la matrice adhésive.

10. Procédé de fabrication d'un article destiné à être collé de manière adhésive sur la peau, comprenant le revêtement d'un substrat de support avec la solution d'adhésif acrylique sensible à la pression selon la revendication 4 et séchage de la solution.

# Figure 1

A                    B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6126865 A
- WO 9703144 A

- WO 02062881 A2